# EUROPEAN PATENT SPECIFICATION

(11) **EP 2 535 060 B1**
(45) Date of publication and mention of the grant of the patent: **12.02.2014**
(21) Application number: 11170462.3
(22) Date of filing: 17.06.2011
(51) Int. Cl.: A61L 15/18

(54) **Absorbent articles comprising hydratable non-deliquescent inorganic salts**
Saugfähige Artikel mit hydratisierbaren, nicht zerfließbaren anorganischen Salzen
Articles absorbants comprenant des sels inorganiques hydratables non déliquescents

(43) Date of publication of application: 19.12.2012
(73) Proprietor: The Procter & Gamble Company, Cincinnati, OH 45202 (US)
(72) Inventor: Carlucci, Giovanni, 66100 Chieti (IT); Ruiz Pardo, Adelaida, 1213 Geneva (CH)
(74) Representative: Briatore, Andrea

(56) References cited:
- US-A1- 2008 221 277

## Description

### FIELD OF THE INVENTION

The present invention relates to absorbent articles comprising a hydratable non-deliquescent inorganic salt to help block blood-containing aqueous fluids, such as for example menses.

### BACKGROUND OF THE INVENTION

Absorbent articles are known in the art. Typical examples for personal hygiene include sanitary napkins, panty liners, adult incontinence articles, infant diapers, paper towels, bath tissue and facial tissue. Such articles are often used to absorb and retain bodily fluids and other exudates excreted by the human body.

Fluids are often retained in absorbent articles within an absorbent element comprising absorbent materials which often include superabsorbent materials, such as absorbent gelling materials (AGM), usually in finely dispersed form, e.g. typically in particulate form. Conventional superabsorbent materials known in the art for use in absorbent articles typically comprise water insoluble, water swellable, hydrogel forming crosslinked absorbent polymers which are capable of absorbing large quantities of liquids and of retaining such absorbed liquids under moderate pressure. In general, absorbent articles comprising conventional absorbent gelling materials commonly have good absorption and retention characteristics to water and urine; however, there still remains room for improvement for absorption and retention towards certain liquids. In particular, proteinaceous or serous body fluids such as typically menses, blood, vaginal secretions, milk, or more particularly blood-containing aqueous fluids require more time to be effectively absorbed and consequently, especially in case of large amount of fluids, these might not be retained by the article and may leak outside.

Therefore in some cases it may be desirable to provide absorbent articles which are able to prevent leakage of body fluids, especially proteinaceous body fluids such as blood-containing aqueous fluids, even in the case when large amounts of fluids are discharged. US2008/0221277A1 describes absorbent polymer structures trated with inorganic salts and oxides.

### SUMMARY OF THE INVENTION

The present invention provides an absorbent article comprising a composition for blocking blood-containing aqueous fluids, wherein the composition comprises one or more hydratable non-deliquescent inorganic salts. Absorbent articles according to the present invention have improved retention properties for blood-containing aqueous fluids.

### DETAILED DESCRIPTION OF THE INVENTION

The term "absorbent article" is used herein in a very broad sense including any article able to receive and/or absorb and/or contain and/or retain fluids and/or exudates, especially bodily fluids/bodily exudates. Exemplary but not exclusively absorbent articles in the context of the present invention are disposable absorbent articles. The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner). Typical disposable absorbent articles according to the present invention are personal hygiene articles such as diapers, surgical and wound dressings and perspiration pads, incontinence pads, as well as absorbent articles for feminine hygiene like sanitary napkins, pantiliners, vaginal tampons, interlabial devices, nursing pads or the like. Absorbent articles suitable for use in the present invention include any type of structures, from a single absorbent layer to more complex multi layer structures. Certain absorbent articles include a fluid pervious topsheet, a backsheet, which may be fluid impervious, and an absorbent core comprised there between.

The term "use", as used herein, refers to the period of time that starts when the absorbent article is actually put in contact with the anatomy of a wearer.

By "body fluid" it is meant herein any fluid produced by human body including, but not limited to, perspiration, urine, menstrual fluids, vaginal secretions and the like. Particularly, by "blood-containing aqueous fluid" it is meant a fluid, typically a body fluid, containing blood, such as for example blood as such and menses.

Hydratable inorganic salts are inorganic salts which can contain water molecules in a defined ratio in their crystal structure. A hydratable inorganic salt is said to be anhydrous when it does not contain any water in its crystal structure. As it is known in chemistry, hydratable inorganic salts are capable of holding water molecules combined in a definite ratio within their crystal structure, forming hydrates. Such hydrates are also said to contain water of crystallization, or water of hydration. The notation of hydrous compound ·*n*H₂O, where *n* is the number of water molecules per formula unit of the salt, is commonly used to show that a salt is hydrated.

Hydrates can normally lose water upon appropriate heating, so providing the corresponding anhydrous salts. Typically, when the water of crystallization is removed, the structure of the hydrate crumbles into an anhydrous powder. Hydratable inorganic salts have different levels of affinity for water, or hygroscopy. Some hydratable inorganic salts have such a strong affinity with water, that they will absorb relatively large amounts of water if they are exposed to it, for example even moisture from the atmosphere, forming a liquid solution. Salts which form a liquid solution by absorbing water are said to be deliquescent. Typical deliquescent salts are for example calcium chloride, magnesium chloride, zinc chloride, potassium carbonate, potassium phosphate, and so on.

Therefore "hydratable non-deliquescent inorganic salts" according to the present description are hydratable inorganic salts which do not possess this characteristic, i.e. upon exposure to water they do absorb it in the definite ratio in order to form the hydrate, but do not form a liquid solution.

The present invention relates to an absorbent article comprising a composition for blocking the blood-containing aqueous fluids, wherein the composition comprises one or more hydratable inorganic salts which are non-deliquescent.

According to the present description, the one or more hydratable non-deliquescent inorganic salts comprised in the composition can be typically anhydrous. Hydratable non-deliquescent inorganic salts to be comprised in absorbent articles according to the present invention can also be selected among those which form hydrates with more than six water molecules, i.e., can hold more than six water molecules in the formula unit, hence in the ·*n*H₂O notation mentioned above, the hydratable non-deliquescent inorganic salts can have *n*>6.

An exemplary hydratable non-deliquescent inorganic salt according to the present description can be magnesium sulfate (MgSO₄), which indeed actually loses the water of hydration to air at ordinary temperatures, and is capable of absorbing water forming at least a heptahydrate (*n*≥7).

In general, according to the present description the absorbent article can also comprise a superabsorbent material.

The absorbent article according to the present description may comprise an absorbent core intended to retain body fluids which may include natural or synthetic absorbent fibers or foams and/or one or more superabsorbent polymers. The absorbent article according to the present description may also typically comprise a fluid pervious topsheet and a fluid impervious backsheet, with the absorbent core comprised therebetween. In these embodiments the composition may be comprised in any or all of topsheet, backsheet or may be comprised within additional elements interposed between them in any way which allow the contact of the composition with fluids upon use of the article. In addition it can also be present into the absorbent core.

The composition may be incorporated in any manner available to the skilled person such as finely dispersed, e.g. in powder form, within the selected element of the absorbent article, such as for example coated or printed on or within the selected element or elements of the absorbent article.

For example, the composition can be provided also to the absorbent core adjacent to a surface thereof, e.g. to the surface which, in use, is opposite to the direction of the incoming fluid, which in a personal hygiene absorbent article such as a sanitary napkin corresponds, in use, to the surface facing the wearer's garment.

The composition can be provided in the absorbent article, namely to the selected element or elements, in a uniform or non-uniform, continuous or non-continuous distribution. For example, according to an aspect of the present description, the composition can be provided only to a peripheral zone of the absorbent core, leaving the inwardly located zone free.

According to an aspect of the present description the absorbent article can also comprise a further substrate layer, typically a fibrous layer, such as for example an airlaid web or a spunlaced web, adjacent to the absorbent core, for example comprised between the absorbent core and the fluid pervious backsheet, wherein the composition can be provided to the substrate layer. The composition can be for example actually comprised between the substrate layer and the absorbent core.

In general, the composition can be comprised in the article according to the present description, being provided to at least a selected element thereof, in order to block blood-containing aqueous fluids within the absorbent article, typically at the selected element comprising the composition.

The amount of composition which is usually present in the absorbent articles according to the present description can be from 10 mg to 10.000 mg per each absorbent article, or from 20 mg to 8.000 mg per each absorbent article, or from 50 mg to 5.000 mg per each absorbent article.

According to an aspect of the present description the absorbent article can be a feminine hygiene article like a sanitary napkin, an interlabial pad, a vaginal tampon or a pantiliner. According to another aspect of the present description, the absorbent article can be a surgical or wound dressing, or also a meat pad.

The composition can be introduced within the absorbent article in any form, including in dry powder form, as a suspension in a liquid or as a solution.

An absorbent article according to the present description has an improved capacity to retain body fluids, particularly blood-containing aqueous body fluids such as typically menses or blood.

Without wishing to be bound by theory it is believed that the composition acts as a dryer for the high content of water present in the blood containing fluid so as to cause a solidification of the overall fluid, actually blocking it within the structure of the absorbent article, and hence an increased retention of the fluid blocked within the absorbent article. The efficiency of this working mechanism can be further enhanced when the composition comprises a hydratable non-deliquescent inorganic salt, such as magnesium sulafate, which typically does not absorb moisture from air, since its absorption capacity can be fully utilized towards the fluid entering the absorbent article.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent article comprising a fluid pervious topsheet, a fluid impervious backsheet, and an absorbent core comprised therebetween, further comprising a substrate layer adjacent to said absorbent core,
said article also comprising a composition for blocking blood-containing aqueous fluids, said composition comprising one or more hydratable non-deliquescent inorganic salts,
wherein said composition is provided to any or all of said topsheet, backsheet and surbstrate layer.

2. An absorbent article according to claim 1, wherein said one or more inorganic salts are anhydrous.

3. An absorbent article according to any preceding claim, wherein said one or more inorganic salts form hydrates with more than six water molecules.

4. An absorbent article according to any preceding claim, wherein said composition comprises magnesium sulfate.

5. An absorbent article according to any preceding claim wherein said composition is comprised in said absorbent core.

6. An absorbent article according to any preceding claim, wherein said substrate layer is an airlaid web or a spunlaced web.

7. An absorbent article according to any preceding claim, wherein said composition is comprised in an amount from 10 mg to 10.000 mg, preferably from 20 mg to 8.000 mg, more preferably from 50 mg to 5.000 mg.

8. An absorbent article according to any preceding claim, further comprising a superabsorbent material.

9. An absorbent article according to any preceding claim, wherein said absorbent article is a sanitary napkin, or a pantiliner, or a vaginal tampon, or an interlabial pad.

10. An absorbent article according to any of claims 1 to 8, wherein said absorbent article is a pad for absorbing blood, such as a surgical or wound dressing or a meat pad.

11. Use of an absorbent article according to any preceding claim for the absorption of blood-containing aqueous fluids, for example menses.

## Patentansprüche

1. Absorptionsartikel, umfassend eine flüssigkeitsdurchlässige Oberschicht, eine flüssigkeitsundurchlässige Unterschicht und einen dazwischen liegenden Absorptionskern, ferner umfassend eine Substratschicht, die an den Absorptionskern angrenzt,
wobei der Artikel auch eine Zusammensetzung zum Abhalten von bluthaltigen, wässrigen Flüssigkeiten umfasst, wobei die Zusammensetzung ein oder mehrere hydratisierbare, nicht zerfließende, anorganische Salze umfasst,
wobei die Zusammensetzung auf einen beliebigen oder alle der Bestandteile Oberschicht, Unterschicht und Substratschicht aufgetragen wird.

2. Absorptionsartikel nach Anspruch 1, wobei das eine oder die mehreren anorganischen Salze wasserfrei sind.

3. Absorptionsartikel nach einem der vorstehenden Ansprüche, wobei das eine oder die mehreren anorganischen Salze Hydrate mit mehr als sechs Wassermolekülen bilden.

4. Absorptionsartikel nach einem der vorstehenden Ansprüche, wobei die Zusammensetzung Magnesiumsulfat umfasst.

5. Absorptionsartikel nach einem der vorstehenden Ansprüche, wobei die Zusammensetzung in dem Absorptionskern enthalten ist.

6. Absorptionsartikel nach einem der vorstehenden Ansprüche, wobei die Substratschicht eine luftgelegte Bahn oder eine wasserstrahlverfestigte Bahn ist.

7. Absorptionsartikel nach einem der vorstehenden Ansprüche, wobei die Zusammensetzung in einer Menge von 10 mg bis 10.000 mg, vorzugsweise von 20 mg bis 8.000 mg, mehr bevorzugt von 50 mg bis 5.000 mg enthalten ist.

8. Absorptionsartikel nach einem der vorstehenden Ansprüche, ferner umfassend ein Superabsorber-Material.

9. Absorptionsartikel nach einem der vorstehenden Ansprüche, wobei der Absorptionsartikel eine Damenbinde oder eine Slipeinlage oder ein Vaginaltampon oder ein Interlabialpad ist.

10. Absorptionsartikel nach einem der Ansprüche 1 bis 8, wobei der Absorptionsartikel ein Pad zum Absorbieren von Blut ist, wie ein chirurgischer Verband oder Wundverband oder ein Fleischpad.

11. Verwendung eines Absorptionsartikels nach einem der vorstehenden Ansprüche zur Absorption von bluthaltigen, wässrigen Flüssigkeiten, zum Beispiel Menstruationsflüssigkeit.

## Revendications

1. Article absorbant comprenant une feuille de dessus perméable aux fluides, une feuille de fond imperméable aux fluides, et une âme absorbante comprise entre elles, comprenant en outre une couche de substrat adjacente à ladite âme absorbante,
ledit article comprenant également une composition pour bloquer des fluides aqueux contenant du sang, ladite composition comprenant un ou plusieurs sels inorganiques hydratables non déliquescents,
dans lequel ladite composition est fournie à l'une quelconque ou toutes parmi lesdites feuille de dessus, feuille de fond et couche de substrat.

2. Article absorbant selon la revendication 1, dans lequel lesdits un ou plusieurs sels inorganiques sont anhydres.

3. Article absorbant selon l'une quelconque des revendications précédentes, dans lequel lesdits un ou plusieurs sels inorganiques forment des hydrates avec plus de six molécules d'eau.

4. Article absorbant selon l'une quelconque des revendications précédentes, dans lequel ladite composition comprend du sulfate de magnésium.

5. Article absorbant selon l'une quelconque des revendications précédentes, dans lequel ladite composition est comprise dans ladite âme absorbante.

6. Article absorbant selon l'une quelconque des revendications précédentes, dans lequel ladite couche de substrat est une nappe appliquée par jet d'air ou une nappe lacée par filage.

7. Article absorbant selon l'une quelconque des revendications précédentes, dans lequel ladite composition est comprise en une quantité allant de 10 mg à 10 000 mg, de préférence de 20 mg à 8000 mg, plus préférablement de 50 mg à 5000 mg.

8. Article absorbant selon l'une quelconque des revendications précédentes, comprenant en outre un matériau superabsorbant.

9. Article absorbant selon l'une quelconque des revendications précédentes, où ledit article absorbant est une serviette hygiénique, ou un protège-slip, ou un tampon vaginal, ou un tampon interlabial.

10. Article absorbant selon l'une quelconque des revendications 1 à 8, où ledit article absorbant est un tampon pour absorber le sang, tel qu'un pansement chirurgical ou pour plaies ou un buvard à viande.

11. Utilisation d'un article absorbant selon l'une quelconque des revendications précédentes, pour l'absorption de fluides aqueux contenant du sang, par exemple, des règles.
